# EUROPÄISCHE PATENTANMELDUNG

(11) **EP 2 238 886 A1**
(43) Veröffentlichungstag der Anmeldung: **13.10.2010**
(21) Anmeldenummer: 10159193.1
(22) Anmeldetag: 07.04.2010
(51) Int. Cl.: A61B 1/04, A61B 5/00

(54) **Vorrichtung zur Fluoreszenzdiagnose**

(30) Priorität: 08.04.2009 DE 102009018141
(71) Anmelder: Karl Storz GmbH & Co. KG, 78532 Tuttlingen (DE)
(72) Erfinder: Beck, Gerd, 78573, Wurmlingen (DE); Ehrhardt, André, 78573, Wurmlingen (DE); Irion, Klaus-Martin, 78576, Emmingen-Liptingen (DE)
(74) Vertreter: Witte, Weller & Partner

(57) **Zusammenfassung**

Eine Vorrichtung zur Fluoreszenzdiagnose weist ein Beleuchtungssystem (12) zum Beleuchten eines Zielgebiets (50), das eine Lichtquelle (16), die dazu ausgelegt ist, Weißlicht zu erzeugen, und einen Beleuchtungspfad (20) aufweist, und ein Beobachtungssystem (14) zum Beobachten des Zielgebiets (50) auf, das einen Beobachtungspfad (36) aufweist, wobei das Beleuchtungssystem (12) einen ersten Beleuchtungsmodus, in dem im Beleuchtungspfad (20) ein Beleuchtungsspektralfilter (54) angeordnet ist, das in einem Anregungsspektralbereich durchlässig und im Spektralbereich auf der langwelligen Seite außerhalb des Anregungsspektralbereichs im Wesentlichen undurchlässig ist, und einen zweiten Beleuchtungsmodus aufweist, in dem das Beleuchtungsspektralfilter (54) nicht im Beleuchtungspfad (20) angeordnet ist, wobei im ersten Beleuchtungsmodus und im zweiten Beleuchtungsmodus im Beobachtungspfad (36) ein Beobachtungsspektralfilter (56) angeordnet ist, das in dem Anregungsspektralbereich im Wesentlichen undurchlässig und im Spektralbereich auf der langwelligen Seite außerhalb des Anregungsspektralbereichs durchlässig ist. Das Beobachtungsspektralfilter (56) ist zusätzlich im Spektralbereich auf der kurzwelligen Seite außerhalb des Anregungsspektralbereichs durchlässig.

## Beschreibung

Die Erfindung betrifft eine Vorrichtung zur Fluoreszenzdiagnose, mit einem Beleuchtungssystem zum Beleuchten eines Zielgebiets, das eine Lichtquelle, die dazu ausgelegt ist, Weißlicht zu erzeugen, und einen Beleuchtungspfad aufweist, und mit einem Beobachtungssystem zum Beobachten des Zielgebiets, das einen Beobachtungspfad aufweist, wobei das Beleuchtungssystem einen ersten Beleuchtungsmodus, in dem im Beleuchtungspfad ein Beleuchtungsspektralfilter angeordnet ist, das in einem Anregungsspektralbereich durchlässig und im Spektralbereich auf der langwelligen Seite außerhalb des Anregungsspektralbereichs im Wesentlichen undurchlässig ist, und einen zweiten Beleuchtungsmodus aufweist, in dem das Beleuchtungsspektralfilter nicht im Beleuchtungspfad angeordnet ist, wobei im ersten Beleuchtungsmodus und im zweiten Beleuchtungsmodus im Beobachtungspfad ein Beobachtungsspektralfilter angeordnet ist, das in dem Anregungsspektralbereich im Wesentlichen undurchlässig und im Spektralbereich auf der langwelligen Seite außerhalb des Anregungsspektralbereichs durchlässig ist.

Eine solche Vorrichtung zur Fluoreszenzdiagnose ist aus EP 0 861 044 B1 (Irion et al.) bekannt.

Eine erfindungsgemäße Vorrichtung zur Fluoreszenzdiagnose der eingangs genannten Art kann zu medizinischen Diagnosezwecken, aber auch zu technischen Diagnosezwecken in industriellen oder wissenschaftlichen Anwendungen verwendet werden. Obwohl die Erfindung hiernach mit Bezug auf die medizinische Fluoreszenzdiagnose beschrieben wird, ist die Erfindung hierauf nicht beschränkt.

Die aus dem eingangs genannten Dokument EP 0 861 044 B1 bekannte Vorrichtung wird zur medizinischen Fluoreszenzdiagnose eingesetzt.

Die medizinische Fluoreszenzdiagnose dient zur Beurteilung des Zustands von biologischem Gewebe, bspw. zur Tumorerkennung, aber auch zur Erkennung der Durchblutung und Vitalität. Mit einer Vorrichtung zur Fluoreszenzdiagnose der eingangs genannten Art kann die Fluoreszenzdiagnose insbesondere in vivo durchgeführt werden.

Eine "fluoreszierende Substanz" im Sinne der vorliegenden Erfindung kann ein Fluoreszenzfarbstoff, ein Pigment usw. sein, die zuvor in das Zielgebiet eingebracht wurde, beispielsweise durch Verabreichen der fluoreszierenden Substanz oder einer Vorstufe desselben an einen Patienten (Mensch oder Tier). Eine "fluoreszierende Substanz" kann jedoch auch eine bereits im Zielgebiet vorhandene Substanz sein, beispielsweise im Fall der medizinischen Fluoreszenzdiagnose eine gewebespezifische Substanz, die durch die Fluoreszenzdiagnose zur Autofluoreszenz angeregt wird. Die vorliegende Erfindung kann beide Fälle umfassen. Für die nachfolgenden Erläuterungen wird von dem Fall ausgegangen, dass die fluoreszierende Substanz ein Fluoreszenzfarbstoff ist, der künstlich in das Zielgebiet, d.h. das zu untersuchende Gewebeareal eingebracht wird.

Dazu wird dem zu untersuchenden Patienten der Fluoreszenzfarbstoff oder eine Ausgangsform dieses Fluoreszenzfarbstoffes verabreicht. In der Fluoreszenzdiagnose zur Gewebsuntersuchung, die mit der bekannten Vorrichtung durchgeführt wird, wird dem Patienten als Ausgangssubstanz 5-Aminolävulinsäure verabreicht, aus der durch Hämbiosynthese intrazellulär der Fluoreszenzfarbstoff Protoporphyrin IX gebildet wird. Bei der Fluoreszenzdiagnose wird ausgenutzt, dass sich der Fluoreszenzfarbstoff, im vorstehenden Fall Protoporphyrin IX, in malignem Gewebe, beispielsweise Tumorgewebe, stärker anreichert als in gesundem Gewebe.

Der Fluoreszenzfarbstoff kann durch Beleuchtung mit Licht in einem Spektralbereich, in dem der Fluoreszenzfarbstoff absorbiert, zur Fluoreszenz angeregt werden. Der Fluoreszenzfarbstoff Protoporphyrin IX absorbiert am stärksten Licht in einem Spektralbereich um 400 nm (Soret-Bande), also im violettblauen sichtbaren Spektralbereich. Das Fluoreszenzlicht ist stets langwelliger als das Anregungslicht, und bei dem Fluoreszenzfarbstoff Protoporphyrin liegt die Fluoreszenzemission im sichtbaren Spektralbereichmit einem Maximum bei 635 nm und einem weiteren Peak bei 705 nm, also im roten sichtbaren Spektralbereich. Die Intensität des Fluoreszenzlichts ist wesentlich geringer als die Intensität des Anregungslichts.

Die bekannte Vorrichtung zur Fluoreszenzdiagnose weist ein Beleuchtungssystem zum Beleuchten des Zielgebiets, d.h. eines Gewebeareals, das auf das Vorhandensein malignen Gewebes untersucht werden soll, und in dem der Fluoreszenzfarbstoff vorhanden ist, und ein Beobachtungssystem zum Beobachten des Gewebeareals auf. Das Beleuchtungssystem weist eine Lichtquelle auf, die dazu ausgelegt ist, Weißlicht zu erzeugen.

Das Weißlicht wird entlang des Beleuchtungspfads in das Gewebeareal gerichtet, um den dort vorhandenen Fluoreszenzfarbstoff zur Fluoreszenz anzuregen. Da das Weißlicht mehr oder weniger kontinuierlich das gesamte Wellenlängenspektrum zumindest des sichtbaren Spektralbereichs enthält, andererseits jedoch nur der Fluoreszenzfarbstoff zur Fluoreszenz angeregt werden soll, ist in dem Beleuchtungspfad des Beleuchtungssystems ein Beleuchtungsspektralfilter angeordnet, das in einem Anregungsspektralbereich, der zumindest teilweise im Absoptionsspektralbereich des Fluoreszenzfarbstoffes liegt, durchlässig und im Spektralbereich auf der langwelligen Seite außerhalb des Anregungsspektralbereichs im Wesentlichen undurchlässig ist. Dadurch wird verhindert, dass von dem Gewebeareal reflektiertes Beleuchtungslicht die deutlich schwächere Fluoreszenz überstrahlt, die, wie oben erwähnt, im Spektralbereich auf der langwelligen Seite außerhalb des Absorptionsspektralbereichs liegt. Im Beobachtungspfad ist ein Beobachtungsspektralfilter angeordnet, das demgegenüber in dem Anregungsspektralbereich im Wesentlichen undurchlässig und im Spektralbereich auf der langwelligen Seite außerhalb des Anregungsspektralbereichs durchlässig ist. Das Beobachtungsfilter ist somit für das im Gewebeareal reflektierte Anregungslicht im Anregungsspektralbereich im Wesentlichen undurchlässig, und lässt demgegenüber das Fluoreszenzlicht zur Beobachtung durch das Auge, ein Beobachtungsinstrument wie ein Endoskop, ein Mikroskop oder eine Kamera durch.

Würde das Beobachtungsspektralfilter gegenüber dem vom Beleuchtungsspektralfilter durchgelassenen Anregungslicht vollkommen undurchlässig sein, könnte durch das Beobachtungssystem nur die von dem Fluoreszenzfarbstoff stammende Fluoreszenz (und ggf. vom Gewebe herrührende unspezifische Fluoreszenz, s.u.) beobachtet werden, die räumlich auf diejenigen Orte im Zielgebiet beschränkt ist, an denen der Fluoreszenzfarbstoff vorhanden ist. In den übrigen Bereichen, in denen der Fluoreszenzfarbstoff nicht vorhanden ist, ist kein Licht durch das Beobachtungssystem zu beobachten, wodurch die Orientierung in dem beobachteten Zielgebiet erschwert bis unmöglich ist. Außerdem ist in der Praxis keine vollständige Trennung der Fluoreszenz des Fluoreszenzfarbstoffes von der Fluoreszenz endogener Fluorochrome zu erreichen, wodurch sich gesundes Gewebe in so aufgenommenen Fluoreszenzbildern dunkelrot darstellt, maligne Gewebeareale entsprechend heller. Es ist jedoch häufig schwierig, in solchen Bildern eine Unterscheidung zwischen gesundem und malignem Gewebe zu treffen.

Aus diesem Grund ist das Beobachtungsspektralfilter an das Beleuchtungsspektralfilter so angepasst, dass das Beobachtungsspektralfilter noch gerade soviel Anregungslicht durchlässt, dass gegenüber der zu beobachtenden spezifischen Fluoreszenz noch schwächere unspezifische Fluoreszenzen überstrahlt und auch die Gewebebereiche, in denen kein Fluoreszenzfarbstoff enthalten ist, mit ausreichender Helligkeit beobachtbar sind, wobei diese im Fall der bekannten Vorrichtung zur Fluoreszenzdiagnose in Bezug auf den Fluoreszenzfarbstoff Protoporphyrin IX blau erscheinen, während die Fluoreszenz rot erscheint.

Die vorstehenden Ausführungen beziehen sich auf den ersten Betriebsmodus des Beleuchtungssystems, der auch als Fluoreszenzmodus bezeichnet wird.

Die bekannte Vorrichtung zur Fluoreszenzdiagnose kann jedoch auch in einem zweiten Betriebsmodus des Beleuchtungssystems betrieben werden, in dem der Beleuchtungsspektralfilter aus dem Beleuchtungspfad entfernt ist, so dass das Weißlicht über den Beleuchtungspfad vollständig in das Zielgebiet gelangt. Dieser Betriebsmodus dient dazu, dem Beobachter eine natürliche Darstellung des beobachteten Zielgebiets zur Verfügung zu stellen. Dieser Betriebsmodus, der auch als Weißlichtmodus bezeichnet wird, sollte eine Beobachtung des Zielgebiets mit möglichst naturgetreuer Farbwiedergabe ermöglichen. Bei der bekannten Vorrichtung zur Fluoreszenzdiagnose kann im Weißlichtmodus das Beobachtungsspektralfilter im Beobachtungspfad belassen werden, da das Beobachtungsspektralfilter der bekannten Vorrichtung im sichtbaren Spektralbereich ab etwa 450 nm durchlässig ist. Das Beobachtungsspektralfilter schneidet also nur den Spektralbereich unterhalb von etwa 450 nm ab, und somit wird nur ein kleiner spektraler Anteil im blauen Spektralbereich des von dem Zielgebiet reflektierenden Lichts verworfen. Das so erhaltene Bild aus dem Zielgebiet entspricht hinsichtlich seiner Farbtreue nahezu dem gewohnten Weißlichtbild, wobei durch Bildverarbeitungsmaßnahmen wie einen Weißabgleich die natürliche Farbdarstellung noch verbessert werden kann.

Die bekannte Vorrichtung zur Fluoreszenzdiagnose lässt sich somit zwischen dem Fluoreszenzmodus und dem Weißlichtmodus umschalten, ohne dass das Beobachtungsspektralfilter aus dem Beobachtungspfad entfernt werden muss. Dies wird bei der bekannten Vorrichtung jedoch durch die "günstige" Lage des Absorptionsspektralbereichs des Fluoreszenzfarbstoffes Protoporphyrin IX ermöglicht.

Andere Fluoreszenzfarbstoffe, die in der medizinischen Diagnostik verwendet werden, haben Absorptionsspektren, die im mittleren oder langwelligen Bereich des sichtbaren Spektralbereichs liegen, wie dies beispielsweise bei dem Fluoreszenzfarbstoff Indocyaningrün und seinen Derivaten, sowie Substanzen mit ähnlichen Eigenschaften der Fall ist. Würde das Konzept der bekannten Vorrichtung auf solche Fluoreszenzfarbstoffe übertragen werden, müsste das kurzwellige Ende des Durchlassbereichs des Beobachtungsspektralfilters zu größeren Wellenlängen hin verschoben werden, wodurch im Weißlichtmodus ein größerer Teil des sichtbaren Spektrums des im Zielgebiet reflektierten Lichts nicht zur naturgetreuen Farbdarstellung des beobachteten Zielgebiets beitragen würde. Der Farbeindruck des beobachteten Zielgebiets wäre dann in nicht akzeptabler Weise verfälscht. Das im Beobachtungspfad im Fluoreszenzmodus vorhandene Beobachtungsspektralfilter müsste im Weißlichtmodus daher aus dem Beobachtungspfad entfernt werden, um ein naturgetreues Weißlichtbild beobachten zu können. Dies würde jedoch durch das Vorsehen eines Filterwechslers den konstruktiven Aufwand bei der Ausgestaltung des Beobachtungssystems und damit die Gestehungskosten der Vorrichtung nachteiligerweise erhöhen.

Aus DE 41 33 493 A1 (Asahi K.K.) ist eine Vorrichtung zur Fluoreszenzdiagnose bekannt, bei der das Beleuchtungssystem im Beleuchtungspfad ein Beleuchtungsspektralfilter aufweist, das im Spektralbereich zwischen 400 nm und 500 nm durchlässig ist. Im Beobachtungspfad des Beobachtungssystems befindet sich ein Beobachtungsspektralfilter, das im Spektralbereich von 500 nm bis 600 nm durchlässig ist. Diese bekannte Vorrichtung kann nur im Fluoreszenzmodus betrieben werden.

Weitere Vorrichtungen zur Fluoreszenzdiagnose sind aus US 6,899,675, US 5,590,660 und US 6,061,591 bekannt.

Der Erfindung liegt daher die Aufgabe zugrunde, eine Vorrichtung zur Fluoreszenzdiagnose der eingangs genannten Art dahingehend weiterzubilden, dass unabhängig von der fluoreszierenden Substanz ein Fluoreszenzbild im Fluoreszenzmodus sowie eine möglichst naturgetreue Farbdarstellung des Zielgebiets im Weißlichtmodus ermöglicht wird, ohne dass das Beobachtungsspektralfilter im Weißlichtmodus aus dem Beobachtungspfad entfernt werden muss.

Erfindungsgemäß wird diese Aufgabe hinsichtlich der eingangs genannten Vorrichtung zur Fluoreszenzdiagnose dadurch gelöst, dass das Beobachtungsspektralfilter zusätzlich in einem Spektralbereich auf der kurzwelligen Seite außerhalb des Anregungsspektralbereichs durchlässig ist.

Die erfindungsgemäße Vorrichtung zur Fluoreszenzdiagnose weist demnach im Beobachtungspfad des Beobachtungssystems ein Beobachtungsspektralfilter auf, das zwei Durchlassbereiche aufweist, und zwar einen ersten Durchlassbereich auf der langwelligen Seite außerhalb des Anregungsspektralbereichs und einen zweiten Durchlassbereich auf der kurzwelligen Seite außerhalb des Anregungsspektralbereichs. Im Anregungsspektralbereich selbst ist das Beobachtungsspektralfilter im Wesentlichen undurchlässig, wobei es sich versteht, dass das Beobachtungsspektralfilter im Anregungsspektralbereich noch eine geringe Resttransmission von beispielsweise weniger als etwa 5% aufweisen kann. Wenn nun der Absorptionsspektralbereich der fluoreszierenden Substanz vollständig innerhalb des sichtbaren Spektralbereichs liegt, hat die erfindungsgemäße Vorrichtung den Vorteil, dass im Weißlichtmodus mit Ausnahme des vom Beobachtungsspektralfilter im Wesentlichen ausgeblendeten Anregungsspektralbereichs weitgehend alle Spektralkomponenten des Weißlichts vom Beobachtungsspektralfilter durchgelassen werden, so dass im Weißlichtmodus, in dem das Beleuchtungsspektralfilter aus dem Beleuchtungspfad entfernt ist, ein bei der Beobachtung des Zielgebiets weitgehend natürlicher Farbeindruck entsteht. Im Fluoreszenzmodus, in dem der Beleuchtungsspektralfilter im Beleuchtungspfad angeordnet ist, kann mit der erfindungsgemäßen Vorrichtung das Fluoreszenzbild mit demselben Beobachtungsspektralfilter, der im Weißlichtmodus verwendet wird, übertragen werden. Bei der erfindungsgemäßen Vorrichtung kann somit das Beobachtungsspektralfilter sowohl im Fluoreszenzmodus als auch im Weißlichtmodus im Beobachtungspfad verbleiben, wodurch der konstruktive Aufwand der erfindungsgemäßen Vorrichtung wesentlich verringert bzw. vereinfacht ist. Beispielsweise kann auf einen Wechsler zum Wechseln, Herausnehmen oder Einbringen des Beobachtungsspektralfilters im Beobachtungssystem verzichtet werden. Hierdurch ist auch ein wesentlich schnelleres Umschalten des Beobachtungssystems zwischen dem Fluoreszenzmodus und dem Weißlichtmodus möglich.

Die erfindungsgemäße Vorrichtung zur Fluoreszenzdiagnose ist nicht auf eine bestimmte fluoreszierende Substanz beschränkt. Es gilt für die erfindungsgemäße Vorrichtung, dass das erfindungsgemäße Prinzip auch auf andere fluoreszierende Substanzen übertragbar ist. Allgemein gilt, dass eine in Betracht gezogene fluoreszierende Substanz eine Absorption in einem Absorptionsspektralbereich zwischen zwei spektralen Grenzen Y1 und Y2 aufweist, wobei die Naturkonstanten Y1 und Y2 vorzugsweise in vivo gemessen werden. Die spektralen Grenzen Y1 und Y2 können beide oder teilweise im sichtbaren Spektralbereich liegen. Das Beobachtungsspektralfilter ist im Sinne der vorliegenden Erfindung auf der kurzwelligen Seite der spektralen Grenze Y1 bis zu einer spektralen Grenze Y1-ΔX durchlässig und auf der langwelligen Seite ab einer spektralen Grenze Y2+ΔX. ΔX kann dabei im Bereich von etwa 15 nm bis etwa 45 nm liegen, wobei ΔX je nach technischer Realisierbarkeit von Spektralfiltern so klein wie möglich sein sollte.

Des Weiteren sollte der vom Beobachtungsspektralfilter ausgeblendete Anregungsspektralbereich, in dem das Beleuchtungsspektralfilter durchlässig ist, so schmal wie möglich sein. So ist in einer bevorzugten Ausgestaltung vorgesehen, dass der Anregungsspektralbereich etwa gleich breit oder schmaler als ein Absorptionsspektralbereich einer fluoreszierenden Substanz im Zielgebiet ist.

Auf diese Weise kann vorteilhafterweise ein größtmöglicher Teil der sichtbaren Spektralbereiche im Weißlichtmodus für eine farbgetreue Farbwiedergabe des Bildes des Zielgebiets genutzt werden.

In einer weiteren bevorzugten Ausgestaltung ist das Beobachtungsspektralfilter auf der langwelligen Seite außerhalb des Anregungsspektralbereichs zumindest bis in das nahe Infrarot und/oder auf der kurzwelligen Seite außerhalb des Anregungsspektralbereichs bis in den UV-Spektralbereich durchlässig.

Hierbei ist von Vorteil, dass im Weißlichtmodus vom sichtbaren Spektralbereich des im Zielgebiet reflektierten Beleuchtungslichts auch die kurzwelligen und/oder die langwelligen Komponenten zur möglichst naturgetreuen Farbgebung beitragen können.

In einer weiteren bevorzugten Ausgestaltung weist das Beleuchtungsspektralfilter eine erste Transmissionscharakteristik und das Beobachtungsspektralfilter eine zweite Transmissionscharakteristik auf, wobei die erste Transmissionscharakteristik komplementär zur zweiten Transmissionscharakteristik ist.

Diese Maßnahme hat den Vorteil, dass das Beobachtungsspektralfilter eine hohe Transmission bis nahe an den Anregungsspektralbereich aufweisen kann, und zwar sowohl auf der kurzwelligen Seite als auch auf der langwelligen Seite des Anregungsspektralbereichs, wodurch im Weißlichtmodus auch die Spektralkomponenten des reflektierten Beleuchtungslichts bis nahe an den Anregungsspektralbereich heran zur möglichst naturgetreuen Farbbildgebung beitragen. Im Grundsatz gilt, dass das Beobachtungsspektralfilter und das Beleuchtungsspektralfilter hinsichtlich ihrer Transmission optimal an den Anregungsspektralbereich angepasst werden, um einerseits im Fluoreszenzmodus die Fluoreszenz deutlich beobachten zu können, und andererseits im Weißlichtmodus eine möglichst naturgetreue Farbdarstellung zu erhalten.

In einer weiteren bevorzugten Ausgestaltung weist das Beobachtungsspektralfilter im Anregungsspektralbereich eine Transmission im Bereich von etwa 0,05% bis etwa 5% auf.

Diese Maßnahme hat im Fluoreszenzmodus den Vorteil, dass im Fluoreszenzbild der Farbkontrast zwischen der zu beobachtenden spezifischen Fluoreszenz und unspezifischen Fluoreszenzerscheinungen erhöht ist. Dem Fluoreszenzbild wird gerade soviel von dem Beleuchtungslicht im Anregungsspektralbereich beigemischt, dass der Farbkontrast optimal ist. Außerdem wird durch das intensitätsschwach beigemischte Beleuchtungslicht die Orientierung des Betrachters im Fluoreszenzbild verbessert. Auch wird der subjektiv empfundene visuelle Farbkontrast erhöht, was zu einer deutlicheren Unterscheidung des Gewebeareals führt.

Im Rahmen der zuvor genannten Ausgestaltung kann das Beobachtungsspektralfilter eine Transmission im Bereich von etwa 0,05% bis etwa 5% im Anregungsspektralbereich entweder punktuell oder über das gesamte Spektralband des Anregungsspektralbereichs oder in einem oder mehreren Teilbändern des Anregungsspektralbereichs aufweisen.

In einer weiteren bevorzugten Ausgestaltung weist das Beleuchtungsspektralfilter eine erste Transmissionsfunktion auf, die an ihrem kurzwelligen und/oder langwelligen Ende eine Flanke mit einer Steigung zwischen dem Transmissionsminimum und dem Transmissionsmaximum im Bereich von etwa 100%/(45 nm) bis etwa 100%/(10 nm) aufweist.

Die Verwendung eines Beleuchtungsspektralfilters, dessen Transmissionsfunktion an ihrem kurzwelligen und/oder langwelligen Ende eine steile Flanke aufweist, hat den Vorteil, dass der Durchlassbereich des Beobachtungsspektralfilters an dessen dem Anregungsspektralbereich benachbarten langwelligen Ende und/oder an dessen dem Anregungsspektralbereich benachbarten kurzwelligen Ende sehr nahe an den Durchlassbereich des Beleuchtungsspektralfilters heranreichen kann, was im Weißlichtmodus wiederum zur verbesserten Farbdarstellung beiträgt, ohne dass jedoch die Durchlassbereiche des Beobachtungsspektralfilters in den Durchlassbereich des Beleuchtungsspektralfilters hineinreichen, wodurch die intensitätsärmere Fluoreszenz im Fluoreszenzmodus kontraststark beobachtet werden kann.

In gleicher Weise ist es bevorzugt, wenn das Beobachtungsspektralfilter eine zweite Transmissionsfunktion aufweist, die an ihrem der ersten Transmissionsfunktion benachbarten kurzwelligen und/oder langwelligen Ende eine Flanke mit einer Steigung zwischen dem Transmissionsminimum und dem Transmissionsmaximum im Bereich von etwa 100%/(45 nm) bis 100%/(10 nm) aufweist.

Eine maximal technisch realisierbare Steilheit der Flanken der ersten Transmissionsfunktion und der zweiten Transmissionsfunktion ist für die vorliegende Erfindung bevorzugt. Damit lassen sich die Durchlassbereiche des Beobachtungsspektralfilters und des Anregungsspektralfilters mit minimalem spektralen Abstand der Flanken zueinander bei gleichzeitig hoher Trennschärfe zwischen diesen Durchlassbereichen aneinander anpassen.

In einer weiteren bevorzugten Ausgestaltung ist ein spektraler Abstand zwischen einem 50%-Transmissionswert des Beobachtungsspektralfilters und einem 50%-Transmissionswert des Beleuchtungsspektralfilters am kurzwelligen und/oder langwelligen Ende des Anregungsspektralbereichs kleiner als etwa 45 nm, vorzugsweise kleiner als etwa 20 nm, und beträgt weiter vorzugsweise etwa 15 nm.

Diese Maßnahme ist insbesondere in Kombination mit den vorstehend beschriebenen Ausgestaltungen von Vorteil, wonach die Transmissionsfunktionen des Beleuchtungsspektralfilters und des Beobachtungsspektralfilters eine steile lineare Flanke aufweisen. Mit einem derart geringen spektralen Abstand der 50%-Transmissionswerte der Flanken der Transmissionsfunktionen des Beobachtungsspektralfilters und des Beleuchtungsspektralfilters wird eine hinreichende Trennung der Durchlassbereiche bei gleichzeitig geringem spektralen Abstand der Durchlassbereiche voneinander erreicht.

In bevorzugten Ausgestaltungen liegt der 50%-Transmissionswert des Beobachtungsspektralfilters am langwelligen Ende des Spektralbereichs SB-B1 im Bereich von etwa 640 nm bis etwa 740 nm, weiter vorzugsweise im Bereich von etwa 660 nm bis etwa 720 nm.

In weiteren bevorzugten Ausgestaltungen liegt der 50%-Transmissionswert des Beobachtungsspektralfilters am langwelligen Ende des Spektralbereichs SB-B1 im Bereich von etwa 720 nm bis etwa 780 nm, weiter vorzugsweise im Bereich von etwa 730 nm bis etwa 760 nm.

In noch weiteren bevorzugten Ausgestaltungen liegt der 50%-Transmissionswert des Beobachtungsspektralfilters am langwelligen Ende des Spektralbereichs SB-B1 im Bereich von etwa 660 nm bis etwa 720 nm, weiter vorzugsweise im Bereich von etwa 670 nm bis etwa 710 nm.

In weiteren bevorzugten Ausgestaltungen liegt der 50%-Transmissionswert des Beobachtungsspektralfilters am kurzwelligen Ende des Spektralbereichs SB-B2 im Bereich von etwa 780 nm bis etwa 850 nm, weiter vorzugsweise im Bereich von etwa 800 nm bis etwa 830 nm.

In noch weiteren bevorzugten Ausgestaltungen liegt der 50%-Transmissionswert des Beobachtungsspektralfilters am kurzwelligen Ende des Spektralbereichs SB-B2 im Bereich von etwa 790 nm bis etwa 840 nm, weiter vorzugsweise im Bereich von etwa 800 nm bis etwa 830 nm.

In noch weiteren bevorzugten Ausgestaltungen liegt der 50%-Transmissionswert des Beobachtungsspektralfilters am kurzwelligen Ende des Spektralbereichs SB-B2 im Bereich von etwa 740 nm bis etwa 810 nm, weiter vorzugsweise im Bereich von etwa 750 nm bis etwa 780 nm.

Der Anregungsspektralbereich SB-A, in dem das Beleuchtungsspektralfilter durchlässig ist, ist entsprechend an die jeweils gewählten Durchlassbereiche des Beobachtungsspektralfilters angepasst.

Die Transmissionscharakteristika des Beobachtungsspektralfilters und des Anregungsspektralfilters, insbesondere die vorstehend genannten 50%-Transmissionswerte, werden in Abhängigkeit der in Betracht gezogenen fluoreszierenden Substanz gewählt. Die vorliegende Erfindung ist jedoch auf keine bestimmte fluoreszierende Substanz beschränkt.

In einer weiteren bevorzugten Ausgestaltung ist im ersten Betriebsmodus im Beleuchtungspfad zusätzlich ein IR-Spektralfilter angeordnet.

Hierbei ist von Vorteil, dass der Wärmeeintrag durch das Beleuchtungslicht in das Zielgebiet reduziert wird.

In entsprechender Weise kann im zweiten Betriebsmodus im Beleuchtungspfad vorzugsweise zusätzlich ein IR-Spektralfilter angeordnet sein.

Bei beiden vorstehend genannten Maßnahmen kann vorteilhafterweise eine Lichtquelle verwendet werden, die Weißlicht mit einer hohen Lichtintensität emittiert, wobei das Weißlicht auch deutliche Infrarot-Anteile wie bei dem Licht einer Glühlampe, Kurzbogenlampe oder dgl. besitzen kann, während die zusätzlich vorgesehenen IR-Spektralfilter verhindern, dass eine übermäßige Wärmestrahlung in das Zielgebiet geleitet wird.

In weiteren bevorzugten Ausgestaltungen weist das Beobachtungssystem ein Endoskop oder ein Mikroskop auf.

Hierbei ist von Vorteil, dass sowohl die Lichtzuführung entlang des Beleuchtungspfades als auch die Rückführung des Beobachtungslichts aus dem Zielgebiet gemeinsam in das Endoskop oder das Mikroskop integriert werden können, wodurch ein von dem Beobachtungsinstrument getrenntes Lichtzuführungsinstrument nicht erforderlich ist. Das Endoskop oder Mikroskop kann mit einer oder ohne eine Kamera ausgestattet sein.

In einer weiteren bevorzugten Ausgestaltung weist das Beobachtungssystem eine Kamera auf, die im sichtbaren und im daran angrenzenden nahen Infrarot empfindlich ist, und die mit dem Beleuchtungssystem über eine Kommunikationsschnittstelle, insbesondere einen Feldbus, mit dem Beleuchtungssystem verbunden ist, über die der erste bzw. zweite Betriebsmodus synchronisiert wird.

Hierbei ist von Vorteil, dass das Umschalten zwischen dem Fluoreszenzmodus und dem Weißlichtmodus vom Benutzer der Vorrichtung bspw. über die Kopftasten der Kamera gesteuert werden kann. "Synchronisiert" bedeutet hier, dass die Betriebszustände von Kamera und Beleuchtungssystem automatisch aneinander angeglichen bzw. angepasst werden. Die Kamera kann für den IR-Bereich einen separaten für den IR-Bereich optimierten Bildaufnehmer aufweisen.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine Vorrichtung zur Fluoreszenzdiagnose in einer schematischen Darstellung;
- Fig. 2: ein Diagramm von Transmissionscharakteristiken eines Beobachtungsspektralfilters und eines Beleuchtungsspektralfilters in einer Prinzipdarstellung;
- Fig. 3: ein Diagramm einer Transmissionscharakteristik eines Ausführungsbeispiels eines Beleuchtungsspektralfilters;
- Fig. 4: ein Diagramm einer Transmissionscharakteristik eines Ausführungsbeispiels eines Beobachtungsspektralfilters; und
- Fig. 5: ein Diagramm einer gemeinsamen Darstellung der Transmissionscharakteristiken des Beobachtungsspektralfilters und des Beleuchtungsspektralfilters gemäß den Ausführungsbeispielen in Fig. 3 und 4.

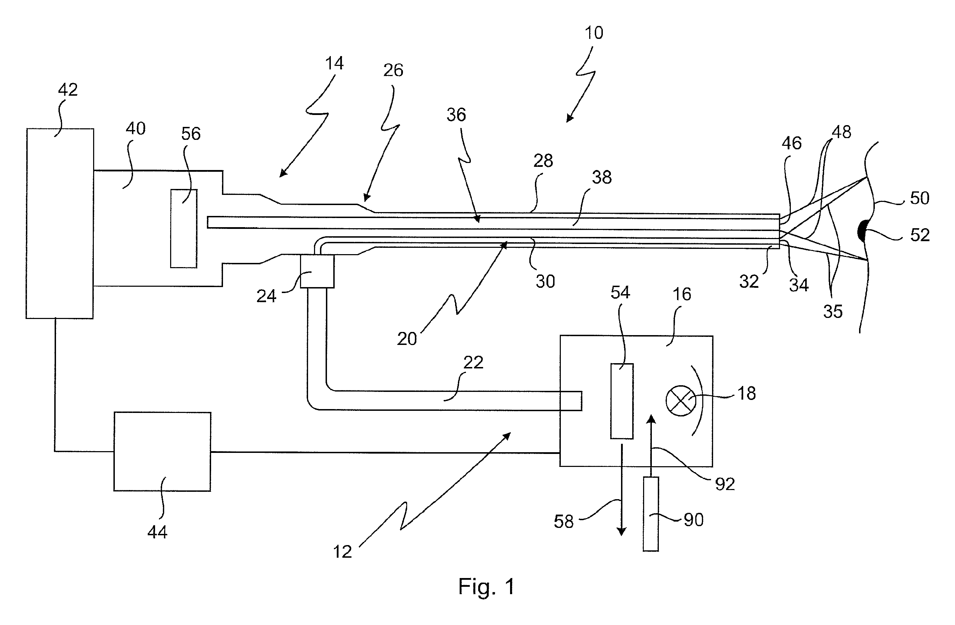

In Fig. 1 ist eine mit dem allgemeinen Bezugszeichen 10 versehene Vorrichtung zur Fluoreszenzdiagnose schematisch dargestellt. Die Vorrichtung 10 wird ohne Beschränkung der Allgemeinheit nachfolgend anhand einer Verwendung zur medizinischen Fluoreszenzdiagnose beschrieben. Die Vorrichtung 10 kann jedoch auch zur technischen Fluoreszenzdiagnose für industrielle oder wissenschaftliche Zwecke eingesetzt werden.

Die Vorrichtung 10 weist allgemein ein Beleuchtungssystem 12 und ein Beobachtungssystem 14 auf.

Das Beleuchtungssystem 12 weist eine Lichtquelle 16 auf, die dazu ausgelegt ist, Weißlicht zu erzeugen. Dazu weist die Lichtquelle 16 eine Lampe 18 oder ein Lampensystem auf, beispielsweise eine Xenon-Entladungslampe. Es können jedoch auch andere Weißlicht erzeugende Lampen oder Lampensysteme verwendet werden, wie beispielsweise Bogenentladungslampen, Glühlampen, LED-Lampensysteme und dergleichen.

Unter "Weißlicht" im Sinne der vorliegenden Erfindung ist polychromatisches bzw. spektral breitbandiges Licht zu verstehen, das ein kontinuierliches oder quasi-kontinuierliches Spektrum zumindest über den sichtbaren Spektralbereich aufweist.

Das Beleuchtungssystem 12 weist weiterhin einen Beleuchtungspfad 20 auf. In dem gezeigten Ausführungsbeispiel ist der Beleuchtungspfad 20 ausgehend von der Lichtquelle 16 teilweise durch ein Lichtleitkabel 22 realisiert, das an einen Lichtleiteranschluss 24 eines Endoskops 26 angeschlossen ist. Das Endoskop 26 weist einen lang erstreckten Schaft 28 auf, durch den sich der Beleuchtungspfad 20 weiter in Form eines Lichtleiters 30, beispielsweise in Form eines Glasfaserbündels, ausgehend von dem Lichtleiteranschluss 24 zu einem distalen Ende 32 des Schafts 28 erstreckt. Über eine Lichtaustrittsfläche 34 tritt das Licht gemäß einem Lichtaustrittskegel 35 aus, wenn die Lichtquelle 16 eingeschaltet ist.

Der Beleuchtungspfad 20 ist somit teilweise in das Endoskop 26 integriert.

Das Beobachtungssystem 14 weist einen Beobachtungspfad 36 auf, der ebenfalls zumindest teilweise in das Endoskop 26 integriert ist. Der Beobachtungspfad 36 kann in dem Endoskop 26 auf verschiedene Weisen realisiert sein, beispielsweise durch ein geordnetes Glasfaserbündel 38, durch ein Relais-Linsensystem, oder im Fall der Ausgestaltung des Endoskops 26 als Videoendoskop durch einen beispielsweise im Bereich des distalen Endes 32 angeordneten Bildsensor, wobei dann die Bildweiterleitung durch elektrische Signalleitungen, die durch den Schaft 28 verlaufen, zu einem proximalen Ende 40 des Endoskops erfolgt.

Anstelle des Endoskops 26 kann die Vorrichtung 10 auch ein Mikroskop aufweisen.

Im gezeigten Ausführungsbeispiel ist eine Kamera 42 jedoch am proximalen Ende 40 des Endoskops 26 angeschlossen.

Die Kamera 42 ist mit dem Beleuchtungssystem 12 über eine Kommunikationsschnittstelle 44 verbunden. Die Kommunikationsstelle 44 kann einen Feldbus, insbesondere einen CAN-Bus mit CAN-Bus-Protokoll, aufweisen.

Das Beobachtungssystem 14 kann über eine Lichteintrittsfläche 46 am distalen Ende 32 des Schafts 28 des Endoskops 26 Beobachtungslicht empfangen, wie mit einem Lichtkegel 48 angedeutet ist.

Ausgehend von der Lichtquelle 16 wird über den Beleuchtungspfad 20 Beleuchtungslicht, das aus der Lichtaustrittsfläche 34 austritt, auf ein Zielgebiet 50 gerichtet. Das Zielgebiet 50 ist beispielsweise ein Gewebeareal im menschlichen oder tierischen Körper, das auf das Vorhandensein von malignem Gewebe hin untersucht werden soll. Das von dem Zielgebiet 50 kommende Licht tritt über die Lichteintrittsfläche 46 in den Beobachtungspfad 36 des Beobachtungssystems 14 ein und wird von der Kamera 42 erfasst.

Die Vorrichtung 10 kann in zwei Betriebsmoden betrieben werden, und zwar in einem ersten Betriebsmodus, in dem mit dem Beleuchtungssystem 12 eine im Zielgebiet 50 befindliche fluoreszierende Substanz 52 zur Fluoreszenz angeregt und die Fluoreszenz über das Beobachtungssystem 14 beobachtet wird, und in einem zweiten Betriebsmodus, in dem mit dem Beleuchtungssystem 20 das Zielgebiet 50 mit Weißlicht beleuchtet und das so beleuchtete Zielgebiet 50 über das Beobachtungssystem 14 wie in der klassischen Endoskopie beobachtet wird. Der erste Betriebsmodus wird nachfolgend als Fluoreszenzmodus und der zweite Betriebsmodus wird nachfolgend als Weißlichtmodus bezeichnet.

Der Begriff "fluoreszierende Substanz" ist vorliegend allgemein zu verstehen. Der Begriff umfasst Fluoreszenzfarbstoffe, Pigmente und dergleichen, und umfasst grundsätzlich alle Stoffe, die zur Fluoreszenz angeregt werden können. Die fluoreszierende Substanz 52 kann vorliegend eine solche sein, die sich durch Verabreichung einer Ausgangssubstanz an einen Patienten durch einen biochemischen Prozess erst im Gewebe bildet und in bestimmten Gewebebereichen verstärkt anreichert, oder die fluoreszierende Substanz 52 kann unmittelbar in dem Zielgebiet 50 appliziert worden sein. Die fluoreszierende Substanz 52 kann jedoch auch ein körpereigener Stoff sein, der also nicht dem Patienten verabreicht worden ist, und der zur Autofluoreszenz angeregt werden kann.

Damit die Vorrichtung 10 im Fluoreszenzmodus und im Weißlichtmodus arbeiten kann, weist das Beleuchtungssystem 12 ein Beleuchtungsspektralfilter 54 und das Beobachtungssystem 14 ein Beobachtungsspektralfilter 56 auf.

Das Beleuchtungsspektralfilter 54 kann an einer beliebigen Stelle im Beleuchtungspfad 20 des Beleuchtungssystems 12 angeordnet sein, wobei das Beleuchtungsspektralfilter 54 im gezeigten Ausführungsbeispiel in der Lichtquelle 16 angeordnet ist. Ebenso kann das Beobachtungsspektralfilter 56 an beliebiger Stelle im Beobachtungspfad 36 angeordnet sein, wobei es hier im Bereich des proximalen Endes 40 des Endoskops 26 angeordnet ist. Das Beobachtungsspektralfilter 56 kann insbesondere auch auf der Lichteintrittsseite des Bildsensors der Kamera 42 angeordnet sein. Das Beobachtungsspektralfilter 54 kann als Interferenzfilter ausgebildet sein.

Das Beleuchtungsspektralfilter 54 ist im Fluoreszenzmodus im Beleuchtungspfad 20 angeordnet, während es im Weißlichtmodus aus dem Beleuchtungspfad 20 entfernt ist, wie mit einem Pfeil 58 angedeutet ist. Das Beobachtungsspektralfilter 56 ist dagegen sowohl im Fluoreszenzmodus als auch im Weißlichtmodus im Beobachtungspfad 36 des Beobachtungssystems 14 angeordnet. Dies wird durch die nachfolgend noch zu beschreibende Ausgestaltung des Beobachtungsspektralfilters 56 ermöglicht.

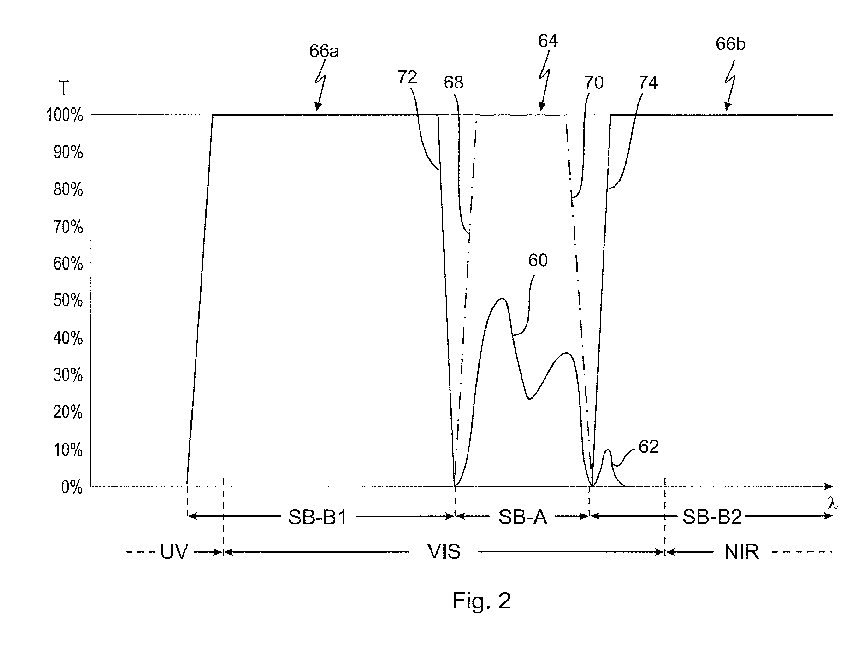

Mit Bezug auf Fig. 2 werden zunächst allgemein die Transmissionscharakteristika des Beobachtungsspektralfilters 56 und des Beleuchtungsspektralfilters 54 beschrieben.

Fig. 2 zeigt ein nicht maßstabsgetreues Diagramm, in dem auf der Abszisse die Wellenlänge λ und auf der Ordinate die Transmission T aufgetragen ist.

In Fig. 2 ist mit VIS der sichtbare Spektralbereich bezeichnet, dessen kurzwelliges Ende bei etwa λ = 380 nm und dessen langwelliges Ende bei etwa λ = 750 nm liegt. Auf der kurzwelligen Seite des sichtbaren Spektralbereichs VIS schließt sich der Ultraviolett-Spektralbereich UV an, und auf der langwelligen Seite des sichtbaren Spektralbereichs VIS schließt sich der Spektralbereich des nahen Infrarot NIR an.

Die Lichtquelle 16 erzeugt, wie bereits oben beschrieben, Weißlicht, das sich entsprechend aus dem kontinuierlichen Spektrum des sichtbaren Spektralbereichs VIS zusammensetzt, wobei das Weißlicht auch Spektralkomponenten im UV-Spektralbereich als auch im NIR-Spektralbereich aufweisen kann.

Eine Absorptionskurve 60 in Fig. 2 stellt das Absorptionsspektrum der fluoreszierenden Substanz 52 dar. Ein Anregungsspektralbereich SB-A des Beleuchtungsspektralfilters 54 liegt in einem Spektralbereich, in dem die fluoreszierende Substanz 52 maximal absorbiert oder der einen geeigneten Teilbereich des Absorptionsspektrums der fluoreszierenden Substanz 52 erfasst, und liegt hier im sichtbaren Spektralbereich VIS. Eine Kurve 62 in Fig. 2 stellt das Fluoreszenzspektrum der fluoreszierenden Substanz 52 dar, wobei das Fluoreszenzspektrum auf der langwelligen Seite teilweise außerhalb des Anregungsspektralbereichs SB-A liegt. Der Anregungsspektralbereich SB-A ist nicht notwendigerweise mit dem Absorptionsspektralbereich der fluoreszierenden Substanz identisch, sondern kann auch breiter oder schmaler sein als der Absorptionsspektralbereich.

Das Beleuchtungsspektralfilter 54 weist eine Transmissionscharakteristik auf, die in Fig. 2 durch eine strichpunktiert dargestellte Transmissionsfunktion 64 repräsentiert ist. Das Beleuchtungsspektralfilter 54 ist gemäß der Transmissionsfunktion 64 im Anregungsspektralbereich SB-A durchlässig, d.h. weist dort eine hohe Transmission T auf, während das Beleuchtungsspektralfilter 54 sowohl auf der langwelligen Seite außerhalb des Anregungsspektralbereichs SB-A als auch auf der kurzwelligen Seite außerhalb des Anregungsspektralbereichs SB-A im Wesentlichen undurchlässig ist, d.h. in diesen Spektralbereichen eine Transmission von etwa 0% aufweist, wobei aus technischen Gründen eine Transmission von 0% nicht erreichbar ist, jedoch eine Transmission von beispielsweise weniger als 0,1%.

Das Beobachtungsspektralfilter 56 weist eine Transmissionscharakteristik auf, gemäß der das Beobachtungsspektralfilter 56 zwei Durchlassbereiche aufweist. Ein erster Durchlassbereich ist in Fig. 2 durch eine Transmissionsteilfunktion 66a und ein zweiter Durchlassbereich durch eine Transmissionsteilfunktion 66b repräsentiert.

Gemäß der Transmissionsteilfunktion 66a ist das Beobachtungsspektralfilter 56 in einem Spektralbereich SB-B1 auf der kurzwelligen Seite außerhalb des Anregungsspektralbereichs SB-A durchlässig, d.h. weist in dem Spektralbereich SB-B1 eine hohe Transmission auf. Der Spektralbereich SB-B1 erstreckt sich vom kurzwelligen Ende des Anregungsspektralbereichs SB-A bis zum oder in den UV-Spektralbereich.

Gemäß der Transmissionsteilfunktion 66b weist das Beobachtungsspektralfilter 56 in einem Spektralbereich SB-B2 auf der langwelligen Seite außerhalb des Anregungsspektralbereichs SB-A ebenfalls eine hohe Transmission auf. Der Spektralbereich SB-B2 erstreckt sich vom langwelligen Ende des Anregungsspektralbereichs SB-A bis zum oder in den NIR-Spektralbereich.

Im Anregungsspektralbereich SB-A ist das Beobachtungsspektralfilter 56 im Wesentlichen undurchlässig, d.h. das Beobachtungsspektralfilter 56 weist im Anregungsspektralbereich SB-A eine Transmission von nahe 0% auf, wobei, wie später noch beschrieben wird, eine geringe Resttransmission von weniger als 5% des Beobachtungsspektralfilters 56 im Anregungsspektralbereich SB-A vorteilhaft ist bzw. sein kann.

Das Beobachtungsspektralfilter 56 weist gemäß Fig. 2 somit nicht nur eine hohe Transmission auf der langwelligen Seite des Anregungsspektralbereichs SB-A auf, sondern auch auf der kurzwelligen Seite des Anregungsspektralbereichs SB-A und weist dort eine hohe Transmission über den sichtbaren Spektralteilbereich bis zum UV-Spektralbereich oder in diesen hinein auf. Dadurch kann das Beobachtungsspektralfilter 56 auch im Weißlichtmodus im Beobachtungspfad verbleiben.

Soweit vorstehend der Begriff "hohe Transmission" verwendet wurde, so ist darunter eine Transmission von über 50%, vorzugsweise von bis zu näherungsweise 100% zu verstehen, wobei eine Transmission von 100% technisch nicht realisierbar ist, jedoch eine Transmission, die sehr nahe an 100% heranreicht, z.B. größer oder gleich 90% oder größer oder gleich 95% ist.

Wie aus Fig. 2 weiter hervorgeht, sind die durch die Transmissionsfunktionen 66a, 66b und 64 repräsentierten Transmissionscharakteristika des Beleuchtungsspektralfilters 54 und des Beobachtungsspektralfilters 56 komplementär zueinander ausgebildet.

Die Transmissionsfunktion 64 des Beleuchtungsspektralfilters 54 weist im Wesentlichen ein trapezförmiges Profil auf, das am kurzwelligen Ende eine Flanke 68 und am langwelligen Ende eine Flanke 70 aufweist, die vom Transmissionsminimum bei etwa 0% bis zum Transmissionsmaximum bei etwa 100% steil ansteigen. Die Steigung der Flanken 68, 70 liegt dabei im Bereich von etwa 100%/(45 nm) bis 100%/(10 nm), wobei die technisch maximal erreichbare Flankensteilheit für die Transmissionsfunktion des Beleuchtungsspektralfilters 54 bevorzugt ist.

Ebenso weist die Transmissionsfunktion 66a, 66b des Beobachtungsspektralfilters 56 ein Profil auf, das an dem der Flanke 68 der Transmissionsfunktion 64 des Beleuchtungsspektralfilters 54 benachbarten kurzwelligen Ende eine Flanke 72 und an dem der Flanke 70 der Transmissionsfunktion 64 des Beleuchtungsspektralfilters 54 benachbarten Ende eine Flanke 74 aufweist, wobei die Flanken 72 und 74 eine Steigung zwischen dem Transmissionsminimum von etwa 0% und dem Transmissionsmaximum von etwa 100% im Bereich von etwa 100%/(45 nm) bis 100%/(10 nm) aufweisen. Je größer die Steigung der Flanken 68, 70 bzw. 72 und 74 ist, desto geringer kann der spektrale Abstand zwischen den Flanken 72 und 68 und den Flanken 70 und 74 gewählt werden, wodurch die Transmissionsbereiche des Beobachtungsspektralfilters 56 in den Spektralbereichen SB-B1 und SB-B2 sehr nahe an den Anregungsspektralbereich SB-A herangeführt werden können. Dadurch gehen im Weißlichtmodus so wenig Spektralkomponenten wie möglich verloren, so dass weitgehend ein natürliches Farbbild im Weißlichtmodus erhalten werden kann.

Die dargestellte Linearität der Flanken 68, 70, 72, 74 ist hier nur eine vereinfachte zeichnerische Darstellung der möglichst hohen Flankensteilheit, die für die Erfindung bevorzugt ist.

Im Fluoreszenzmodus ist das Beleuchtungsspektralfilter 54 im Beleuchtungspfad 20 angeordnet. Von dem von der Lichtquelle 16 erzeugten Weißlicht wird nur das Spektrum im Anregungsspektralbereich SB-A über den Beleuchtungspfad 20 auf das Zielgebiet 50 appliziert. Dabei wird die fluoreszierende Substanz 52 zur Fluoreszenz angeregt, wobei der Beobachtungsspektralfilter 56 das Fluoreszenzspektrum gemäß der Kurve 62 in Fig. 2 durchlässt, während das vom Zielgebiet 50 reflektierte Beleuchtungslicht von dem Beobachtungsspektralfilter 56 im Wesentlichen nicht durchgelassen wird. Wie bereits zuvor erwähnt, weist jedoch das Beobachtungsspektralfilter 56 im Anregungsspektralbereich SB-A eine geringe Resttransmission von weniger als 5% auf, so dass das Beobachtungsspektralfilter 56 eine geringe Menge an Beleuchtungslicht aus dem Anregungsspektralbereich SB-A ebenfalls durchlässt, wodurch der Farbkontrast zwischen der zu beobachtenden Fluoreszenz der fluoreszierenden Substanz 52 und den übrigen Bereichen im Zielgebiet 50 und die Orientierung im Fluoreszenzbild verbessert wird.

Im Weißlichtmodus wird das Beleuchtungsspektralfilter 54 aus dem Beleuchtungspfad 20 entfernt, so dass nunmehr das gesamte Spektrum des Weißlichts über den Beleuchtungspfad 20 in das Zielgebiet 50 gelangt und dieses mit Weißlicht ausgeleuchtet wird. Vom Beobachtungsspektralfilter 56 wird das im Zielgebiet 50 reflektierte Weißlicht bis auf das Spektrum des Weißlichts im Anregungsspektralbereich SB-A durchgelassen, so dass, wenn der Anregungsspektralbereich SB-A schmal ist, aus dem Weißlicht nur ein geringer Spektralbereich ausgeblendet wird, wodurch eine noch tolerierbar natürliche Farbbilddarstellung des Zielgebiets 50 mit der Kamera 42 ermöglicht wird. Zur Verbesserung des Farbeindruckes kann noch ein Weißabgleich durch entsprechende Bildverarbeitungsmaßnahmen vorgenommen werden.

Für die Transmissionscharakteristiken des Beleuchtungsspektralfilters 54 und des Beobachtungsspektralfilters 56 ist eine Optimierung im Hinblick auf einerseits eine ausreichende Anregung der fluoreszierenden Substanz 52 zur Fluoreszenz und einer gut wahrnehmbaren Fluoreszenzdarstellung im Fluoreszenzmodus und im Hinblick auf andererseits eine möglichst naturgetreue Farbdarstellung des beobachteten Zielgebiets 50 im Weißlichtmodus vorzunehmen. Dies wird nachfolgend anhand der spektralen Lage der Flanken 70 und 74 einerseits und der Flanken 68 und 72 andererseits beschrieben.

Eine Verlagerung der Flanken 70 und 74 zu größeren Wellenlängen hin bewirkt, dass die beobachtete Fluoreszenzintensität abnimmt, weil das Beobachtungsspektralfilter 56 dann nur noch einen Teil des Fluoreszenzspektrums durchlässt. Dies ist insbesondere dann kritisch, wenn der Fluoreszenzspektralbereich sehr nahe am Anregungsspektralbereich SB-A liegt, wie in Fig. 2 dargestellt ist. Umgekehrt hat eine Verlagerung der Flanken 70 und 74 zu kürzeren Wellenlängen hin zur Folge, dass das Beleuchtungsspektralfilter 54 einen Anteil des Beleuchtungslichts im Anregungsspektralbereich SB-A ausschneidet, der zur Anregung der Fluoreszenz benötigt wird. Aus diesen Gründen ist die Lage der Flanken 70 und 74 der Transmissionsfunktionen des Beleuchtungsspektralfilters 54 und des Beobachtungsspektralfilters 56 am langwelligen Ende des Anregungsspektralbereichs SB-A in Abhängigkeit von der fluoreszierenden Substanz 52 zu wählen.

Wenn der Anregungsspektralbereich SB-A bereits sehr nahe am langwelligen Ende des sichtbaren Spektralbereichs VIS liegt, hat dagegen eine Verschiebung der Flanken 70 und 74 zu größeren oder kleineren Wellenlängen auf die naturgetreue Farbdarstellung des Zielgebiets 50 im Weißlichtmodus keine oder nur geringe Auswirkungen. Wenn jedoch der Anregungsspektralbereich SB-A tiefer im sichtbaren Spektralbereich VIS liegt, hat eine Verschiebung der Flanken 70 und 74 auch eine Auswirkung auf die Farbtreue des durch das Beobachtungssystem 14 beobachteten Zielgebiets 50, weil durch die Verschiebung der Flanke 74 für die naturgetreue Farbwiedergabe erforderliche Spektralkomponenten dann ausgeblendet werden könnten. Dies wird nachfolgend anhand der Flanken 68 und 72 näher erläutert.

Eine Verschiebung der Flanken 68 und 72 zu höheren Wellenlängen hin hat den Nachteil, dass das für die Anregung der Fluoreszenz im Anregungsspektralbereich SB-A benötigte Beleuchtungslichtspektrum reduziert wird. Dies verschlechtert die Fluoreszenzdarstellung im Fluoreszenzmodus. Wenn der Anregungsspektralbereich SB-A wie in Fig. 2 dargestellt nahe am langwelligen Ende des sichtbaren Spektralbereichs VIS liegt, hat jedoch eine Verschiebung der Flanken 68, 72 zu höheren Wellenlängen hin den Vorteil, dass das Beobachtungsspektralfilter 56 aus dem Anregungsspektralbereich SB-A weniger Spektralkomponenten ausblendet, wodurch die Naturtreue der Farbwiedergabe verbessert wird. Für die Lage der Flanken 68, 72 muss daher ein Optimum gefunden werden, das beiden zuvor genannten Wirkungen der Verschiebung der Flanken 68, 72 Rechnung trägt.

Ein solches Optimum kann mit Hilfe der L*a*b*-Farbmetrik nach der Norm ISO 12647 und ISO 13655 gefunden werden. Die daraus ermittelbare Farbabweichung ΔE stellt ein Maß dafür dar, wie groß die Farbabweichung eines Bildes durch die Filterung durch das Beobachtungsspektralfilter 56 gegenüber einem Bild ist, bei dem das Weißlicht vollständig vom Beobachtungssystem 14 erfasst wird. Dabei ist weiterhin zu berücksichtigen, dass die Farbabweichung sich speziell im Fall von Körpergewebe, insbesondere Schleimhaut, anders verhält als beispielsweise bei dem Bild einer weißen Fläche.

Das Optimum der Lage der Flanken 68, 72 sollte der Bedingung genügen, dass die Farbabweichung ΔE nicht größer als 4,0 ist, was vom menschlichen Auge zwar als Farbunterschied wahrgenommen wird, der jedoch noch tolerierbar ist.

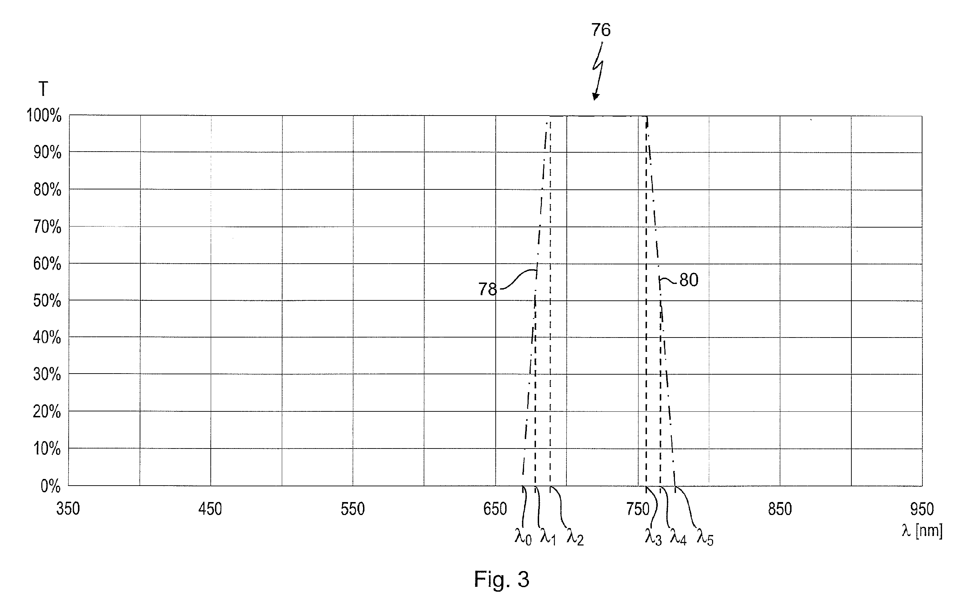

Mit Bezug auf Fig. 3 bis 5 wird nachfolgend ein Ausführungsbeispiel von Transmissionscharakteristika des Beleuchtungsspektralfilters 54 und des Beobachtungsspektralfilters 56 beschrieben.

Fig. 3 zeigt eine schematische Transmissionsfunktion 76 des Beleuchtungsspektralfilters 54. Bei λ₀ = 667,5 nm ist die Transmission T des Beleuchtungsspektralfilters 54 noch etwa 0 %, bei λ₁ = 675 nm beträgt die Transmission T etwa 50%, und bei λ₂ = 682,5 nm ist die Transmission T etwa 100%. Der Anstieg der Transmissionsfunktion 76 vom Transmissionsminimum bei λ₀ zum Transmissionsmaximum bei λ₂ ist steil und weist eine Steigung von 100%/(15 nm) auf. Von λ₂ bis λ₃ = 757,5 nm bleibt die Transmission T des Beleuchtungsspektralfilters 54 maximal, beträgt bei λ₄ = 765 nm noch etwa 50%, und sinkt bei λ₅= 772,5 nm auf etwa 0 % ab. Ebenso wie die Flanke 78 weist die Flanke 80 somit eine Steigung von etwa 100%/(15 nm) auf. Die dargestellte Linearität der Flanken 78, 80 ist hier wiederum nur eine zeichnerische Vereinfachung.

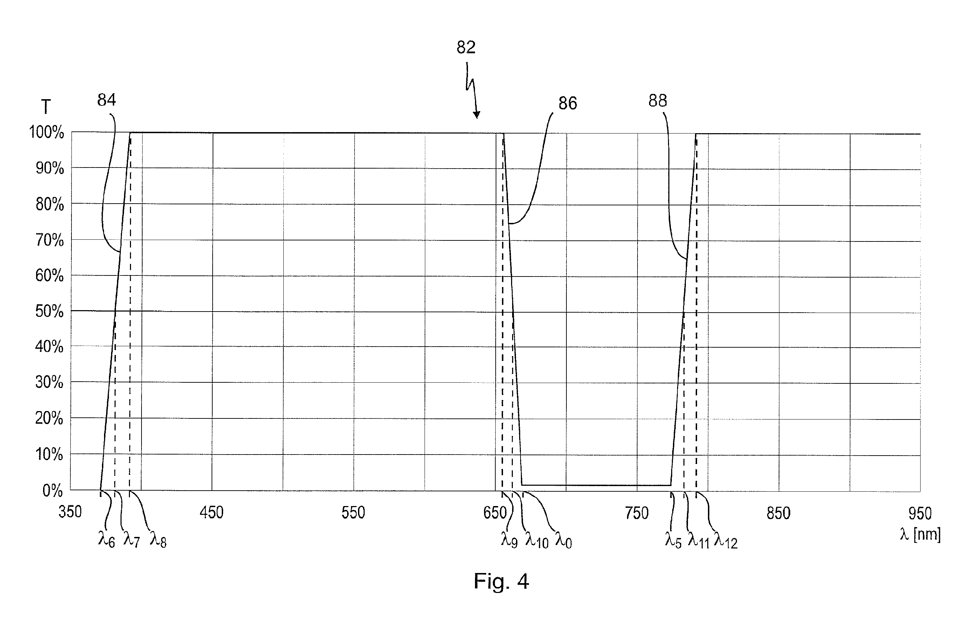

Fig. 4 zeigt eine schematische Transmissionsfunktion 82 des Beobachtungsspektralfilters 56. Die Transmission T des Beobachtungsspektralfilters 56 ist bei λ₆ = 375 nm noch etwa 0%, beträgt bei λ₇ = 382,5 nm etwa 50%, und erreicht bei λ₈ = 390 nm etwa 100%. Bis λ₉ = 652,5 nm bleibt die Transmission T des Beobachtungsspektralfilters 56 maximal, fällt dann entlang einer Flanke 86 bei λ₁₀ = 660 nm auf etwa 50% und bei λ₀ = 667,5 nm auf 2% ab. Im Spektralbereich von λ₀ = 667,5 nm bis λ₅ = 772,5 nm, also in dem Durchlassbereich des Beleuchtungsspektralfilters 54, beträgt die Transmission T des Beobachtungsspektralfilters 56 etwa 2%. Entlang einer weiteren Flanke 88 steigt die Transmission bei λ₁₁ = 780 nm wieder auf etwa 50% und erreicht bei λ₁₂ = 787,5 nm wieder den Maximalwert von etwa 100%, der hier bis 950 nm bestehen bleibt.

Die mittlere Steigung der Flanken 84, 86 und 88 beträgt in diesem Beispiel jeweils 100%/(15 nm).

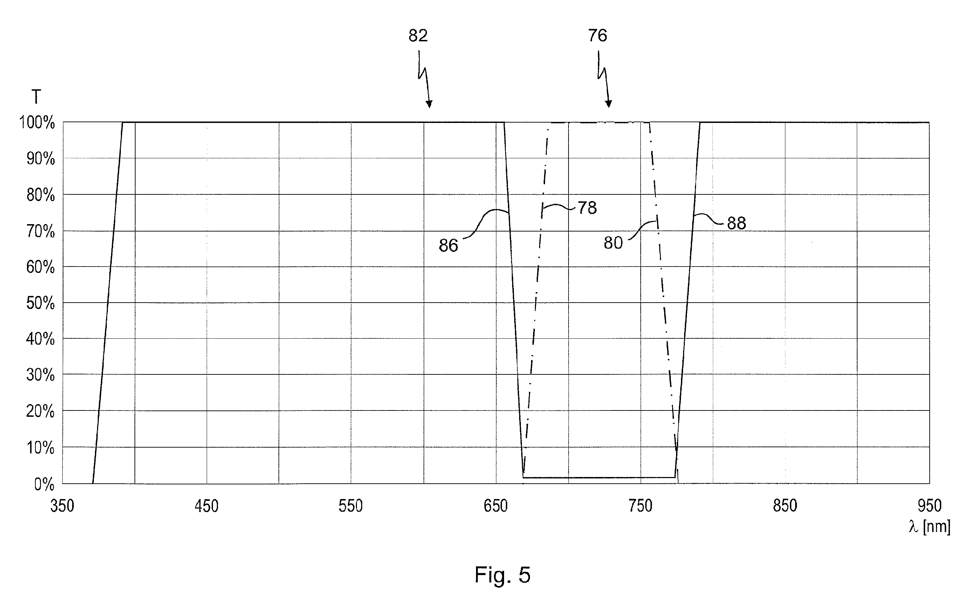

Fig. 5 zeigt die beiden Transmissionsfunktionen 76 und 82 in einem gemeinsamen Diagramm, aus dem sich entnehmen lässt, dass die Transmissionsfunktionen 76 des Beleuchtungsspektralfilters 54 und 82 des Beobachtungsspektralfilters 56 zueinander komplementär sind. Im Spektralbereich von λ = 667,5 nm bis λ = 772,5 nm weist das Beleuchtungsspektralfilter 54 eine hohe Transmission und das Beobachtungsspektralfilter 56 eine geringe Resttransmission von etwa 2% auf, so dass im Fluoreszenzmodus ein Teil des für die Anregung der fluoreszierenden Substanz 52 verwendeten Beleuchtungslichtes dem Fluoreszenzbild beigemischt wird.

Bei den Lagen der Flanken 86 und 78 der Transmissionsfunkionen 76 und 82 ergibt sich eine Farbabweichung ΔE von weniger als 3. Im Weißlichtmodus erscheint das beobachtete Gewebe dann leicht grünlich-bläulich verfärbt, was jedoch tolerierbar ist. Durch einen Weißabgleich kann die Farbverfälschung noch weiter reduziert werden.

Wie sich aus Fig. 3 bis 5 ergibt, haben die 50 %-Transmissionswerte der Transmissionsfunktionen 76 und 82 am kurzwelligen Ende und am langwelligen Ende des Durchlassbereichs des Beleuchtungsspektralfilters 54 einen spektralen Abstand von etwa 15 nm, d.h. die Durchlassbereiche des Beobachtungsspektralfilters 56 liegen beidseitig spektral sehr nahe am Durchlassbereich des Beleuchtungsspektralfilters 54.

Für den Farbstoff Indocyaningrün (ICG) und seine in vivo gebildeten Aggregate, die sowohl Monomere als auch Dimere/Oligomere umfassen, werden nachfolgend Beispiele für die 50%-Transmissionswerte des Beobachtungsspektralfilters (56) am langwelligen Ende des Spektralbereichs SB-B1 (λ₁₀) und am kurzwelligen Ende des Spektralbereichs SB-B2 (λ₁₁) angegeben:

Für den Farbstoff ICG insgesamt wird λ₁₀ im Bereich von etwa 640 nm bis etwa 740 nm, vorzugsweise im Bereich von etwa 660 nm bis etwa 720 nm gewählt. λ₁₁ wird im Bereich von etwa 780 nm bis etwa 850 nm, vorzugsweise im Bereich von etwa 800 nm bis etwa 830 nm gewählt.

Für die im Körper aus ICG gebildeten Monomere wird λ₁₀ im Bereich von etwa 720 nm bis etwa 780 nm, vorzugsweise im Bereich von etwa 730 nm bis etwa 760 nm gewählt. λ₁₁ wird entsprechend im Bereich von etwa 790 nm bis etwa 840 nm, vorzugsweise im Bereich von etwa 800 nm bis etwa 830 nm gewählt.

Für Dimere oder Oligomere, die sich aus ICG im Körper bilden, wird λ₁₀ im Bereich von etwa 660 nm bis etwa 720 nm, vorzugsweise im Bereich von 670 nm bis etwa 710 nm gewählt. λ₁₁ wird entsprechend im Bereich von etwa 740 nm bis 810 nm, vorzugsweise im Bereich von etwa 750 nm bis etwa 780 nm gewählt.

Die 50%-Transmissionswerte des Beleuchtungsspektralfilters 54 am kurzwelligen Ende (λ₁) des Anregungsspektralbereichs SB-A und am langwelligen Ende (λ₄) des Anregungsspektralbereichs SB-A sind in jedem Einzelfall an die gewählten Werte für λ₁₀ und λ₁₁ angepasst.

Wieder mit Bezug auf Fig. 1 ist bei der Vorrichtung 10 weiterhin vorgesehen, dass im Fluoreszenzmodus im Beleuchtungspfad 20 zusätzlich ein IR-Spektralfilter 90 angeordnet sein kann, wie mit einem Pfeil 92 angedeutet ist. Ebenso kann das IR-Spektralfilter 90 oder ein anderes IR-Spektralfilter im Weißlichtmodus im Beleuchtungspfad 20 angeordnet sein. Ein solches IR-Filter 90 kann vermeiden, dass der Wärmeeintrag in den Körper und andere Teile des Systems zu groß wird, indem es IR-Licht entsprechend abblockt. Dabei müssen die Transmissionseigenschaften des IR-Filters 92 jedoch so gewählt werden, dass es im Weißlichtmodus oder im Fluoreszenzmodus die Weißlichtbeobachtung bzw. die Fluoreszenzbeobachtung nicht stört.

Über die Kommunikationsschnittstelle 44, die bspw. in Form eines Feldbusses ausgeführt sein kann, und über die die Kamera 42 mit dem Beleuchtungssystem 12 verbunden ist, kann der Fluoreszenzmodus und der Weißlichtmodus zwischen Kamera und Beleuchtungssystem 12 synchronisiert werden. Die Kommunikation über dem Feldbus kann dabei drahtgebunden oder drahtlos erfolgen.

## Patentansprüche

1. Vorrichtung zur Fluoreszenzdiagnose, mit einem Beleuchtungssystem (12) zum Beleuchten eines Zielgebiets (50), das eine Lichtquelle (16), die dazu ausgelegt ist, Weißlicht zu erzeugen, und einen Beleuchtungspfad (20) aufweist, und mit einem Beobachtungssystem (14) zum Beobachten des Zielgebiets (50), das einen Beobachtungspfad (36) aufweist, wobei das Beleuchtungssystem (12) einen ersten Beleuchtungsmodus, in dem im Beleuchtungspfad (20) ein Beleuchtungsspektralfilter (54) angeordnet ist, das in einem Anregungsspektralbereich (SB-A) durchlässig und im Spektralbereich (SB-B2) auf der langwelligen Seite außerhalb des Anregungsspektralbereichs (SB-A) im Wesentlichen undurchlässig ist, und einen zweiten Beleuchtungsmodus aufweist, in dem das Beleuchtungsspektralfilter (54) nicht im Beleuchtungspfad (20) angeordnet ist, wobei im ersten Beleuchtungsmodus und im zweiten Beleuchtungsmodus im Beobachtungspfad (36) ein Beobachtungsspektralfilter (56) angeordnet ist, das in dem Anregungsspektralbereich (SB-A) im Wesentlichen undurchlässig und im Spektralbereich (SB-B2) auf der langwelligen Seite außerhalb des Anregungsspektralbereichs (SB-A) durchlässig ist, **dadurch gekennzeichnet, dass** das Beobachtungsspektralfilter (56) zusätzlich in einem Spektralbereich (SB-B1) auf der kurzwelligen Seite außerhalb des Anregungsspektralbereichs (SB-A) durchlässig ist.

2. Vorrichtung nach Anspruch 1, **dadurch gekennzeichnet, dass** der Anregungsspektralbereich (SB-A) etwa gleich breit oder schmaler als ein Absorptionsspektralbereich einer fluoreszierenden Substanz (52) im Zielgebiet (50) ist.

3. Vorrichtung nach Anspruch 1 oder 2, **dadurch gekennzeichnet, dass** das Beobachtungsspektralfilter (56) auf der langwelligen Seite außerhalb des Anregungsspektralbereichs (SB-A) zumindest bis in das nahe Infrarot und/oder auf der kurzwelligen Seite außerhalb des Anregungsspektralbereichs (SB-A) bis in den UV-Spektralbereich durchlässig ist.

4. Vorrichtung nach einem der Ansprüche 1 bis 3, **dadurch gekennzeichnet, dass** das Beleuchtungsspektralfilter (54) eine erste Transmissionscharakteristik und das Beobachtungsspektralfilter (56) eine zweite Transmissionscharakteristik aufweist, und dass die erste Transmissionscharakteristik komplementär zur zweiten Transmissionscharakteristik ist.

5. Vorrichtung nach einem der Ansprüche 1 bis 4, **dadurch gekennzeichnet, dass** das Beobachtungsspektralfilter (56) im Anregungsspektralbereich (SB-A) eine Transmission im Bereich von etwa 0,05% bis etwa 5% aufweist.

6. Vorrichtung nach einem der Ansprüche 1 bis 5, **dadurch gekennzeichnet, dass** das Beleuchtungsspektralfilter (54) eine erste Transmissionsfunktion (64; 76) aufweist, die an ihrem kurzwelligen und/oder langwelligen Ende eine Flanke (68, 70; 78, 80) mit einer Steigung zwischen dem Transmissionsminimum und dem Transmissionsmaximum im Bereich von etwa 100%/(45 nm) bis etwa 100%/(10 nm) aufweist.

7. Vorrichtung nach einem der Ansprüche 1 bis 6, **dadurch gekennzeichnet, dass** das Beobachtungsspektralfilter (56) eine zweite Transmissionsfunktion (66a, 66b; 82) aufweist, die an ihrem der ersten Transmissionsfunktion (64; 76) benachbarten kurzwelligen und/oder langwelligen Ende eine Flanke (72, 74; 86, 88) mit einer Steigung zwischen dem Transmissionsminimum und dem Transmissionsmaximum im Bereich von etwa 100%/(45 nm) bis etwa 100%/(10 nm) aufweist.

8. Vorrichtung nach einem der Ansprüche 1 bis 7, **dadurch gekennzeichnet, dass** ein spektraler Abstand zwischen einem 50%-Transmissionswert des Beobachtungsspektralfilters (56) und einem 50%-Transmissionswert des Beleuchtungsspektralfilters (54) am kurzwelligen und/oder langwelligen Ende des Anregungsspektralbereichs (SB-A) kleiner als etwa 45 nm, vorzugsweise kleiner als etwa 20 nm ist, weiter vorzugsweise etwa 15 nm beträgt.

9. Vorrichtung nach einem der Ansprüche 1 bis 8, **dadurch gekennzeichnet, dass** der 50%-Transmissionswert des Beobachtungsspektralfilters (56) am langwelligen Ende des Spektralbereichs (SB-B1) im Bereich von etwa 640 nm bis etwa 740 nm, weiter vorzugsweise im Bereich von etwa 660 nm bis etwa 720 nm liegt.

10. Vorrichtung nach einem der Ansprüche 1 bis 9, **dadurch gekennzeichnet, dass** der 50%-Transmissionswert des Beobachtungsspektralfilters (56) am kurzwelligen Ende des Spektralbereichs (SB-B2) im Bereich von etwa 780 nm bis etwa 850 nm, weiter vorzugsweise im Bereich von etwa 800 nm bis etwa 830 nm liegt.

11. Vorrichtung nach einem der Ansprüche 1 bis 8 oder 10, **dadurch gekennzeichnet, dass** der 50%-Transmissionswert des Beobachtungsspektralfilters (56) am langwelligen Ende des Spektralbereichs (SB-B1) im Bereich von etwa 720 nm bis etwa 780 nm, weiter vorzugsweise im Bereich von etwa 730 nm bis etwa 760 nm liegt.

12. Vorrichtung nach einem der Ansprüche 1 bis 9 oder 11, **dadurch gekennzeichnet, dass** der 50%-Transmissionswert des Beobachtungsspektralfilters (56) am kurzwelligen Ende des Spektralbereichs (SB-B2) im Bereich von etwa 790 nm bis etwa 840 nm, weiter vorzugsweise im Bereich von etwa 800 nm bis etwa 830 nm liegt.

13. Vorrichtung nach einem der Ansprüche 1 bis 8, 10 oder 12, **dadurch gekennzeichnet, dass** der 50%-Transmissionswert des Beobachtungsspektralfilters (56) am langwelligen Ende des Spektralbereichs (SB-B1) im Bereich von etwa 660 nm bis etwa 720 nm, weiter vorzugsweise im Bereich von etwa 670 nm bis etwa 710 nm liegt.

14. Vorrichtung nach einem der Ansprüche 1 bis 9, 11 oder 13, **dadurch gekennzeichnet, dass** der 50%-Transmissionswert des Beobachtungsspektralfilters (56) am kurzwelligen Ende des Spektralbereichs (SB-B2) im Bereich von etwa 740 nm bis etwa 810 nm, weiter vorzugsweise im Bereich von etwa 750 nm bis etwa 780 nm liegt.

15. Vorrichtung nach einem der Ansprüche 1 bis 14, **dadurch gekennzeichnet, dass** das Beobachtungssystem (14) ein Endoskop (26) oder ein Mikroskop aufweist.

16. Vorrichtung nach einem der Ansprüche 1 bis 15, **dadurch gekennzeichnet, dass** das Beobachtungssystem (14) eine Kamera (42) aufweist, die im sichtbaren und im daran angrenzenden nahen Infrarot empfindlich ist, und die mit dem Beleuchtungssystem (12) über eine Kommunikationsschnittstelle (44), insbesondere einen Feldbus, mit dem Beleuchtungssystem verbunden ist, über die der erste bzw. zweite Betriebsmodus synchronisiert wird.
